# EUROPEAN PATENT SPECIFICATION

(11) **EP 1 933 945 B1**
(45) Date of publication and mention of the grant of the patent: **17.03.2010**
(21) Application number: 06795331.5
(22) Date of filing: 25.08.2006
(51) Int. Cl.: A61P 35/00, A61K 31/407

(54) **A PHARMACEUTICAL COMPOSITION COMPRISING MAHANINE USEFUL FOR THE TREATMENT OF PROSTATE CANCER**
PHARMAZEUTISCHE ZUSAMMENSETZUNG VON MAHANIN ZUR BEHANDLUNG VON PROSTATAKREBS
COMPOSITION PHARMACEUTIQUE COMPORTANT DE LA MAHANINE UTILE POUR LE TRAITEMENT DU CANCER DE LA PROSTATE

(30) Priority: 01.09.2005 IN DE23352005
(43) Date of publication of application: 25.06.2008
(73) Proprietor: Council of Scientific and Industrial Research, New Delhi 110 011 (IN)
(72) Inventor: SINHA, Swati, Kolkata 700 032 (IN); PAL, Bikas, Chandra, Kolkata 700 032 (IN); BHATTACHARYA, Samir, West Bengal 731 235 (IN)
(74) Representative: Baldock, Sharon Claire
(86) International application number: PCT/IB2006/002317
(87) International publication number: WO 2007/026203

(56) References cited:
- US-B1- 6 746 694
- DATABASE WPI Week 199646 Derwent Publications Ltd., London, GB; AN 1996-461252 XP002413333 & JP 08 231363 A (LION CORP) 10 September 1996 (1996-09-10)
- DATABASE WPI Week 199747 Derwent Publications Ltd., London, GB; AN 1997-506521 XP002413334 & JP 09 238648 A (INAHATA KORYO KK) 16 September 1997 (1997-09-16)
- SINHA SWATI ET AL: "Mahanine inhibits growth and induces apoptosis in prostate cancer cells through the deactivation of Akt and activation of caspases." THE PROSTATE. 1 SEP 2006, vol. 66, no. 12, 1 September 2006 (2006-09-01), pages 1257-1265, XP002412678 ISSN: 0270-4137 & [Online] Retrieved from the Internet: URL:http://www3.interscience.wiley.com/cgi -bin/abstract/112606119/ABSTRACT> [retrieved on 2006-12-20]
- ROY MOLAY KUMAR ET AL: "Mechanism of mahanine-induced apoptosis in human leukemia cells (HL-60)." BIOCHEMICAL PHARMACOLOGY. 1 JAN 2004, vol. 67, no. 1, 1 January 2004 (2004-01-01), pages 41-51, XP002412706 ISSN: 0006-2952

## Description

### Field of Invention:

The present invention relates to an herbal extract obtained from the leaves or any other plant part of *Murrya koenigii* useful for the treatment of prostate cancer in a subject.

The present invention also relates to a process for the preparation of a pharmaceutical composition comprising an extract obtained from the leaves or any other plant part of *Murraya koenigii* useful for treatment of prostate cancer wherein the said process comprises of homogenizing the dried leaves or any other plant part of *Murraya koenigii* with water wherein the ratio used is 1:1 followed by freeze dried.

More particularly, it relates to a process for isolation of mahanine from *Murraya koenigii.*

It also relates to a new use of the compound mahanine as well as extract obtained from *Murraya koenigii* in the treatment of prostate cancer.

### Background and Prior art of invention:

One of the prevalent forms of neoplasia afflicting men above the age of 65 years is prostate cancer. Mortality from Prostate cancer results from metastasis to bones and lymph nodes. Early detection through serum testing of Prostate Specific Antigen (PSA), improved surgical intervention, radiation therapy, androgen ablation have yielded no answer to the patients who suffer from recurrent or residual cancer (Chinni SR, Li Y, et.al. Indole 3 carbinol(I3C) induced cell growth inhibition , G1 cell cycle arrest and Apoptosis in prostate cancer cells.Oncogene;20:2927-2936(2001). Popular, current therapy of Androgen ablation almost invariably leads to aberrant expression and interaction of Tyrosine kinase and associated ligands. Several recent studies have shown constitutively active mitogenic and cell survival signaling in Prostate cancer. The delicate link between cellular proliferation and apoptosis is challenged by such cellular aberrations. The primary mechanism i.e. prostate carcinoma by-pass apoptosis is by the up regulation of PI3Kinase/Akt survival pathway (Li Y, Sarkar FH. Inhibition of nuclear factor κB activation in PC-3 cells by genistin is mediated via Akt signaling pathway. Clin. Cancer. Res;8: 2369-2377(2002). Akt related serine-threonine in signaling cascade that regulates cell survival are important in the pathogenesis of cancer (Chinni SR, Sarkar FH. Akt inactivation is a key event in Inole 3 carbinol induced apoptosis in PC-3 cells. Clin. Cancer Res; 8: 1228-1236(2002). It inactivates a range of pro apoptotic proteins like Bad, forkhead transcription factor, caspase 9 (Green DR, Reed JC. Mitochondria and Apoptosis. Science; 281: 1309-1312(1998); Thornberry NA, Lazebnik Y. Caspases: enemies within. Science; 281: 1312-1316(1998) while activating Bcl-2 (Adams JM,Cory S. The Bcl-2 protein family Arbiters of cell survival. Science; 281: 1322-1326(1998), NFγB like anti-apoptotic proteins (Datta SR, Brunet A, Greenberg ME. Cellular survival: A play in three Akts. Genes and Dev; 13: 2905-2927(1999). In our endeavour, we isolated, purified mahanine; the active fraction from the leaves of plant *M*. *koenigii(family* rutaceae). Both leaf extracted fraction from *M.Koenigii* and the purified compound mahanine, a carbazole alkaloid shows considerable activity in the induction of apoptosis in two androgen independent Prostate cancer cell lines PC-3 and androgen dependent Prostate cancer cell line LNCaP.

The leaf extract from *M. koenigii* and the purified molecule, mahanine induced apoptosis of androgn independent and androgen dependent prostate cancer cells in a time and dose dependent manner. Leaf extract caused significant apoptosis of all these cell lines (75% cell death in 96 h) with 200µg/ml dosage. The leaf extract from M *koenigii induced* apoptosis of androgen independent and androgen dependent prostate cancer cells in a time and dose dependent manner. Effect of the extract and purified compound on other cells i.e neonatal skeletal muscle cell, cardiomyocyte, hepatocyte have been examined as control cells. This suggests the specificity of the extract and mahanine for inducing apoptosis in prostate cancer cells. In *in vivo* experiment, there was no indication of any toxicity in mahanine treated mice (table 2a, b). Animals were examined for acute toxicity of mahanine; however there was no change in this data till 1 month. Based on this activity the single compound mahanine was purified. It also induced the caspase cascade and down regulation of pAkt as well as Bcl-xl expression, the most important survival signals in cancer cells.

### Objects of the invention:

The main object of the present invention is to provide a pharmaceutical composition useful for the treatment of prostate cancer in a subject.

Another object of the present invention is to provide a new use of the compound mahanine in the treatment of prostate cancer.

Further another object of the present invention is to provide a method of treating prostrate cancer in a subject.

Yet another object of the present invention is to provide a process for the preparation of said pharmaceutical composition comprising an extract obtained from the leaves or any other plant part of *Murraya koenigii* useful for treatment of prostate cancer wherein the said process comprises of homogenizing the dried leaves or any other plant part of *Murraya koenigii* with water wherein the ratio used is 1:1 followed by freeze dried.

Still another object of the present invention is to provide a process for isolation of mahanine from *Murraya* koenigii.

Still another objective of the present invention is to determine cell death, release of cytochrome c, activation caspase cascade, cleavage of PARP DNA repair enzyme, along with the down regulation of Bcl-xl and pAkt, with the extract and the compound mahanine obtained from *Murraya koenigii.*

### Summary of the invention:

The present invention provides use of a composition comprising a therapeutically effective amount of compound mahanine or pharmaceutically acceptable salt thereof optionally along with one or more pharmaceutically acceptable carriers in the manufacture of a medicament for the treatment of prostate cancer, Also provided is Mahanine for use in the treatment of prostate cancer.

Also described is a pharmaceutical composition useful for the treatment of prostate cancer in a subject wherein the said composition comprising the therapeutically effective amount of an extract obtained from any plant parts *Murrya koenigii* or therapeutically effective amount of compound mahanine or its derivatives or analogues or pharmaceutically acceptable salt thereof optionally along with one or more pharmaceutically acceptable carriers. It also relates to a new use of a compound mahanine in the treatment of prostate cancer. Further, described is a process for the preparation of extract and therefore a molecule, mahanine, from the leaf extract or any other plant parts of *Murrya koenigii,* which efficiently kills androgen dependent and independent Prostate cancer cells.

### Brief description of figures:

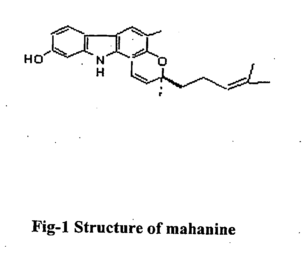

Figure 1 represents structure of Mahanine.

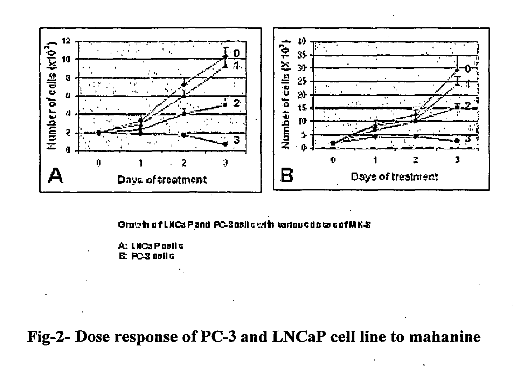

Figure 2 represents dose Response of PC-3 and LNCaP cell line to mahanine. Mahanine killed androgen independent cell line PC-3 and androgen dependent cell line LNCaP cells in a dose and time dependent manner. The dose used was 1,2,3 µg/ml in comparison to control untreared cells (0) in both the cell lines.

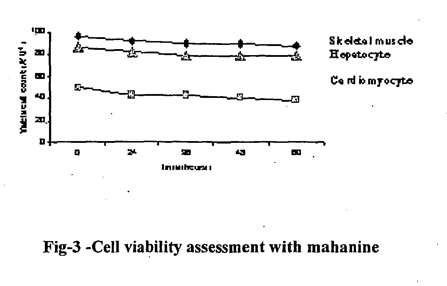

Figure 3 represents cell viability assessment with mahanine. To evaluate the specificity of mahanine, its effect on other cells in primary culture, such as, hepatocytes, cardiomyoctye and skeletal muscle was examined with 9 µg/ml. Mahanine did not kill other cells thereby suggesting its specificity for Prostate cancer cells.

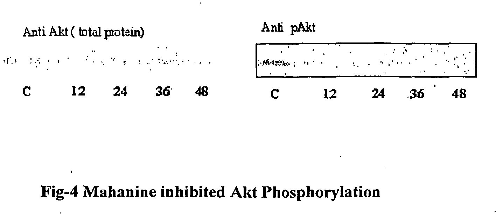

Figure 4 represents mahanine inhibited Akt phosphorylation. Mahanine inhibited phosphorylation of Akt in a dose and time dependent manner. Mahanine showed no effect on the steady state levels of Akt protein but its serine 473 phosphorylation was inhibited at 24 h and by 36 h its activation was totally blocked.

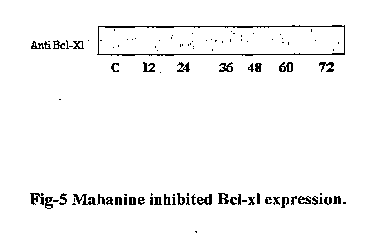

Figure 5 represents inhibition of Bcl-xl expression by mahanine. Mahanine induced apoptosis of PC-3 cells is affected through Bcl-xl. It is an antiapoptotic mitochondrial membrane protein that prevents the release of cytochrome c.Bcl-xl is considerably downregulated by 36 hr thereby inducing apoptosis.

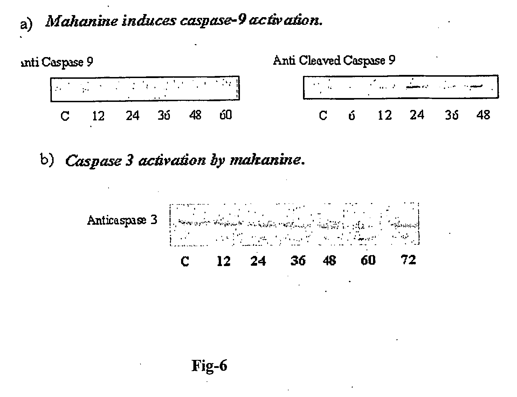

Figure 6(a) represents induction of caspase-9 activation by mahanine. Caspase 9 activation was determined in the same time points with the same dose (3µg/ml) of mahanine. Cleaved caspase 9 could be detected as early as 6h which steadily increased at 12h and 24h and declined at 36h.
Figure 6(b) represents induction of caspase-3 activation by mahanine. The activation of caspase 3 considerably increased at 48h and peak of the activity could be detected at 60h.

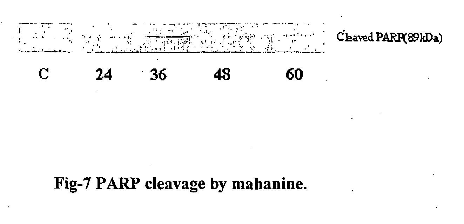

Figure 7 represents PARP cleavage by mahanine. Poly ADP ribosyl Polymerase (PARP) is a DNA repair enzyme responsible for nick detection and repair. It is one of the target proteins of caspase 3. Active caspase 3 causes cleavage of PARP into an 89kDa fragment in PC-3 cells due to mahanine. The maximum cleavage of PARP was observed at 36h.

### Detailed description of the invention:

Presently described a pharmaceutical composition useful for the treatment of prostate cancer in a subject wherein the said composition comprising the therapeutically effective amount of an extract obtained from any plant parts of *Murrya koenigii* or therapeutically effective amount of compound mahanine or its derivatives or analogues or pharmaceutically acceptable salt thereof optionally along with one or more pharmaceutically acceptable carriers.

It is also described ' that the said composition comprises the therapeutically effective amount of an extract obtained from any plant parts of *Murrya koenigii* optionally along with one or more pharmaceutically acceptable carriers.

It is also described that the dosage of the said composition is administered at a unit dose of at least 10-15mg/kg body weight.

It is also described that the said composition further comprises the therapeutically effective amount of compound mahanine or its derivatives or analogues or pharmaceutically acceptable salt thereof optionally along with one or more pharmaceutically acceptable carriers.

The present invention provides use of a composition comprising a therapeutically effective amount of compound mahanine or pharmaceutically acceptable salt thereof optionally along with one or more pharmaceutically acceptable carriers in the manufacture of a medicament for the treatment of prostate cancer.

In another embodiment of the present invention, the mahanine used is obtained from the extract *Murrya Koenigii* or synthetically made.

In still another embodiment of the present invention, the dosage of the said composition is administered at a unit dose of at least 0.1-5mg /kg body weight. In still another embodiment of the present invention, the dosage of the said composition is administered preferably in water-soluble form.

In still another embodiment of the present invention, the said carriers are selected in such a manner that it does not interfere with the activity of traction of Murrya koenigii extract.

In still another embodiment of the present invention, wherein the said carriers is selected from the group consisting of proteins, carbohydrates, sugar, talc, magnesium stearate, cellulose, calcium carbonate, and starch-gelatin paste and pharmaceutical acceptable carriers, excipient, diluent or solvent

In still another embodiment of the present invention, the administration route is selected from the group consisting of oral, intravenous, intramuscular or subcutaneous route.

In still another embodiment of the present invention, the said form for oral route is selected from the group consisting of capsule, syrup, concentrate, powder and granule

In still another embodiment of the present invention, the androgen independent cell line PC 3 and androgen dependent cell line LNCaP is killed by the said composition in a dose and time dependent manner.

Also described is a method of treating prostrate cancer in a subject, wherein the said method comprising the step of administering to the subject a pharmaceutical composition comprising the therapeutically effective amount of an extract obtained from any plant parts of *Murrya koenigii* or therapeutically effective amount of compound mahanine or its derivatives or analogues or pharmaceutically acceptable salt thereof optionally along with one or more pharmaceutically acceptable carriers.

In still another embodiment of the present invention, other cells such as hepatocytes, cardiomayocytes, and skeletal muscle are not killed by the said composition. Also described but not claimed is a process for the preparation of an pharmaceutical compostion comprising an extract obtained from the leaves or any other plant part of *Murraya koenigii* useful for treatment of prostate cancer wherein the said process comprises of homogenizing the dried leaves or any other plant part of *Murraya koenigii* with water wherein the ratio used is 1:1 followed by freeze dried.

Also described is that the plant parts are selected from the group consisting of leaves, sterns, fruits, or any other part of the Murrya Koenigii In another embodiment of the present invention, the leaves of *Murraya koenigii* is collected from different areas of West Bengal, India.

Also described is that the leaves used are taken from fresh or/ and sun shade dried leaves of Murrya Koenigii In still another embodiment of the present invention, the anti-carcinogenic activity of the said extract is confirmed by in-vivo experiments.

Also described is that the use of the said extract is in the treatment of prostate cancer.

Also described is a process for the isolation of compound mahanine from *Murraya* koenigii, wherein the said process comprising the steps of:
a) extracting the fresh leaves of *Murraya* koenigii with methanol;
b) concentrating the methanol extract obtained from step (a) to obtain a residue I;
c) suspending the residue I obtained from step (b) in water followed by extraction with ethyl acetate and n-butanol.
d) concentrating the ethyl acetate layer obtained from step (c) to get a residue II;
e) chromatograph the ethyl acetate fraction obtained from step (d) on silica gel using petroleum ether-chloroform (100:00 → 00:100) as an eluent to give four fraction;
f) subjecting the fraction 4 obtained from step (e) to chromatograpby on silica gel followed by crystallization in petrol to get a desired product.

The structure of the said compound may be
confirmed by comparing its physical data as well as its infrared, NMR and mass spectral data with those of an authentic sample.

The anti-carcinogenic activity of the said compound may be confirmed by in-vivo experiments.

The use of the compound mahanine may be in the treatment of prostate cancer.

Example 1 to 15 relates the extract obtained from *Murraya koenigii* (Rutaceae) and its biological activity thereof.

### Example 1

### Source of Murraya koenigii (Rutaceae)

The leaves of *Murraya koenigii* (Rutaceae) was collected from different areas of West Bengal, India. A voucher specimen has been deposited at the Department of Medicinal Chemistry, Indian Institute of Chemical Biology, Kolkata, India.

### Example 2

### Isolation of extract of Murraya koenigii (Rutaceae)

The fresh leaves and all other plant parts of *Murraya koenigii* (1.2Kg) was homogenized with water (1.5lit) in a mixture-blender and freeze dried. Activity of the freeze-dried material was examined on prostate cancer cell line.

### Example 3

### Culturing of Human prostate cancer cell line, PC-3

Human prostate cancer cell line, PC-3 (PTEN -ve, androgen independent) from American Type Culture Collection, Manassas,VA,USA. Cells were grown in Dulbecco's Modified Eagle Medium supplemented with 10% fetal bovine serum and 1% antibiotic-antimycotic. Cells were cultured in 37°C in an atmosphere of 5% CO₂.

### Primary culture of rat neonatal cardiomyocytes

The cardiomyocytes from 2-day-old neonatal rat were isolated by the method previously described by Yoshihiro Kimura (1994) with modifications. Briefly, heart was excised and minced in pre warmed (37°C) Ads buffer (1.2M NaCl, 198mM HEPES, 54mM KCl, 8.3mM MgSO₄, 55.4mM glucose, 95mM NaH₂PO₄), digested in typeII Collagenase 0.05% and Pancreatin in 3-succesive digestions of 15 minutes each. Supernatant was pooled and cells palleted at 2000g, 10minutes. Cells were resuspended and plated in collagen coated T-25 flaks in Medium-199 enriched with 10% Fetal Bovine Serum and 1% antibiotic-antimycotic.

### Primary culture of rat neonatal hepatocyte

The hepatocytes from 2-day-old neonatal rat were isolated by method described previously by William E Russell (1997) with modifications. Briefly, the livers were perfused through the portal vein with a calcium-free solution consisting of 150 mM NaCl, 2.8 mM KCl, 5.5 mM glucose, and 25 mM HEPES (pH 7.6) for 10 min, followed by mincing and digestion in DMEM containing 0.05% collagenase type IV for 30 minutes. The cells were centrifuged (2000g, 10minutes) and dispersed in DMEM enriched with 10% fetal Bovine Serum, 1% antibiotic-antimycotic.

### Primary culture of neonatal rat skeletal muscle

The skeletal muscles from 2-day-old neonatal rat were isolated by method described previously by William E Russell (1997) with modifications. Briefly, Soleus muscle were dissected, minced and digested with 0.2% Type II Collagenase and 0.05% trypsin in Phosphate buffered Saline (PBS) pH 7.4,0.15M NaCl. The dispersed skeletal muscle cells were centrifuged (1000g, 10minutes) washed and resuspended in Phosphate buffered Saline (PBS) with 1% antibiotic-antimycotic. Cells were preincubated for 30 minutes at 37°C, 95%air/5%CO2. The floating muscle cells were plated on collagen coated T-25 flaks in Dulbecco's modified Eagle Medium enriched with 10% Fetal Bovine Serum and 1% antibiotic-antimycotic

### Example 4

Viability of PC-3 after treatment as at the indicated times was determined by MTT assay -Cell titre 96 *AQ*oueus One solution Cell Proliferation Assay (Promega,Corp.Madison,WI) as per manufacturer's protocol. Briefly, 10,000 cells were plated in triplicate in 96 well plates and incubated for 12 hours in complete media. Compound MK-3 was added as indicated on replacing them to fresh complete media and incubated upto 96hours for varied time periods. 20µl per well of cell titre 96 *AQ*oueus One solution reagent. Incubate the plate for 1-4 hours at 37°C and humidified 5%CO₂ atmosphere. Absorbance was recorded at 490nm with 96 well plate readers. Reference wavelength of 630nm was used to reduce background contributed by nonspecific absorbance due to cell debris.

### Example 5

### Electrophoresis and Immunoblotting

PC-3 cells (1X10⁶ cells) were incubated with complete medium. Cells were detached using cell dissociation reagent from Sigma Chemical company St Louis,MO,USA. They were collected by centrifugation at 1500g for 10 mins and boiled for 5-7 minutes in sodium dodecyl sulphate(SDS) buffer (pH 6.8). Aliquots containing 60µg total cellular protein were separated by 10%SDS-PAGE and transferred to PVDF membrane (MILIPORE, Bedford, MA, USA). Membrane was blocked with blocking buffer for 1hour at room temperature and probed with desired primary antibody caspase-9, Bcl-xl, pAkt1/2/3(ser 473), anti PARP, Cytochrome C (Santa-Cruz Biotechnology, Inc. USA). Anti Caspase -3 that recognizes procaspse-3 (32 kDa) and the active cleaved caspase-3 (17 kDa) (BD Biosciences, Mountainview, CA). Akt, cleaved caspase-9 (Cell Signaling technology, Beverly,MA) overnight at 4° C followed by alkaline phosphatase conjugated secondary antibody and detection.

### Example 6

To search anti-carcinogenic activity from medicinal plants of India, we selected *Murraya koenigii* leaves extract and observed efficient killing of androgen independent prostate cancer cell line PC-3. It shows the data of PC-3 cell line.

### Extract of M.koenigii reduces PC-3 cell viability by inducing Apoptosis

Extracts did not show any toxic effects on neonatal skeletal muscle cell, cardiomyocyte, hepatocyte with the same dose i.e 100µg/ml *M. koenigii* total amounting to of 200µg/ml of culture media suggesting the specificity of the combined extracts for inducing apoptosis in prostate cancer cells.

### In-vivo experiment to confirm in-vitro results

Experiments for acute toxicity are performed as per the guidelines of WHO. Experimental subject: Mice, Balb/C Male, female. 25gms body weight (approx) 16 mice were divided into 2 groups 8 animals were received the dosage of @2gm/kg body weight of combined extract. Equal volume of the vehicle (DMSO) were given to 8 animals wherein dose administered orally once and then animals sacrificed and haematological parameters checked 15 days after treatment.

**Table 1: Haematological tests for crude extract:**

| | RBC | WBC | Hb(gm/dl) |
|---|---|---|---|
| Control | 1029 | 92 | 10.69 |
| Treated | 1032 | 89 | 10.713 |

There was no indication of any toxicity in treated mice (table 1)

### Example 7

### Inhibition of cell-survival pathway bi-herbal extract obtained from M.koenigii treated PC-3 cells

In cell survival pathway, Akt kinase plays an important role by inhibiting apoptotic processes. Akt phosphorylates downstream effector molecules such as pro-apoptotic protein bad effecting its inactivation. This does not permit its dimerisation with Bcl-xl that result in the inhibition of apoptotic process. The bi-herbal extract inhibited phosphorylation of Akt in a dose and time dependent manner. It showed no effect on the steady state levels of Akt protein but its serine 473 phosphorylation was inhibited at 24 h and by 36 h its activation was totally blocked. The basal level of phosphorylation at serine 473 is predominant in PC-3 cell line. The inactivation of Akt phosphorylation could be attributed to the inactivation of cell survival pathways resulting subsequent induction of apoptosis in treated PC-3 cells.

It has been then investigated whether mahanine bi-herbal extract obtained from M.koenigii) induced apoptosis of PC-3 cells is effected through Bcl-xl. Bcl-xl is a mitochondrial membrane protein that maintains mitochondrial membrane integrity in survival signal pathway. The bi-herbal extract significantly decreased Bcl-xl expression at 36 h.

This indicates initiation of apoptotic pathway as down regulation of Bcl-xl permits disintegration of mitochondrial outer membrane that causes leakage of cytochrome c initiating the caspase cascade. The activation of caspase 3 was detected at 48h and peak of the activity could be detected at 60h.

### Example 8

Therapeutic Evaluation of the preparation developed by combination of *M.koenigii* leaves extract for the treatment of prostate cancer (Conducted by registered Auyrvedic practioner). 3 capsules per day (each containing 330mg) were administered orally.

| Patient | weight of prostate before treatment | weight of prostate after 2 months of treatment |
|---|---|---|
| A | 39gm | 21gm |
| B | 46gm | 25gm |
| C | 34 gm | 18 gm |
| D | 42gm | 27 gm |
| E | 29gm | 16gm |
| F | 41gm | 19gm |

All the above patients reported easy flow of urine during both day and night after taking capsules for 2 months

### Example 9

### Isolation of compound Mahanine

The leaves of *Murraya koenigii* (Rutaceae) were collected from different areas of West Bengal, India. A voucher specimen has been deposited at the Department of Medicinal Chemistry, Indian Institute of Chemical Biology, Kolkata. The fresh leaves *of Murraya koenigii* (3Kg) were extracted with methanol in a mixture blender. The methanol extract was concentrated to dryness to give a residue (188g), which was suspended in water and successively extracted with ethyl acetate and n-butanol. The ethyl acetate layer was concentrated to residue (70g). The ethyl acetate extract was tested for bioactivity. A part of ethyl acetate fraction (16g) was chromatographed on silica gel (250g) using petroleum ether-chloroform (100:00 → 00:100) as an eluent to give four fractions, fr.1 -4, in order of elution. Fraction 4 (1.5g) was subjected to repeated chromatography on silica gel and finally crystallization in petrol afforded a compound (0.15g), m.p. 94 -95°C, [α]_{D} +31 (chloroform) identical to the structure of mahanine ( Fig. 1). Its identity was confirmed by comparing its physical data as well as its infrared (IR), ¹H NMR, ¹³C NMR and mass spectral data with those of an authentic sample (Tian-shung Wu, Murrayamine A,B,C and (+) mahanine, carbazole alkaloid from Murraya euchrestifolia, Phytochemistry, 30, 1048 (1991).The mahanine was tested for bioactivity.

### Example 10

The fresh leaves and all other plant parts of *Murrava koenigii* (1.2Kg) was homogenized with chloroform (1.5lit) in a mixture-blender and then sonicated in an ultrasonic bath with 3 burst each for 15 min and allow it for extraction over night. Filtering through Whatman No. 1 filter paper separated the chloroform-extracted material. This process of extraction was repeated three times. The combined extract was evaporated to dryness in a flash evaporator under reduced pressure at 40°C. The residual substance was then dried under high vacuum. The chloroform extract (14g) was chromatographed on silica gel column as described in example-9. Mahanine was isolated as pure crystals (0.12g) and was tested for bioactivity.

### Example 11

Human prostate cancer cell line, PC-3 (PTEN -ve, androgen independent) from American Type Culture Collection, Manassas,VA,USA. Cells were grown in Dulbecco's Modified Eagle Medium supplemented with 10% fetal bovine serum and 1% antibiotic-antimycotic. Cells were cultured in 37°C in an atmosphere of 5% CO₂.

### Primary, culture of rat neonatal cardiomyocytes

The cardiomyocytes from 2-day-old neonatal rat were isolated by the method previously described by Yoshihiro Kimura (1994) with modifications. Briefly, heart was excised and minced in pre warmed (37 degree C) Ads buffer (1.2M NaCl, 198mM HEPES, 54mM KCl, 8.3mM MgSO₄, 55.4mM glucose, 95mM NaH₂PO₄), digested in typeII Collagenase 0.05% and Pancreatin in 3-succesive digestions of 15 minutes each. Supernatant was pooled and cells palleted at 2000g, 10minutes. Cells were resuspended and plated in collagen coated T-25 flaks in Medium-199 enriched with 10% Fetal Bovine Serum and 1% antibiotic-antimycotic.

### Primary culture of rat neonatal hepatocyte

The hepatocytes from 2-day-old neonatal rat were isolated by method described previously by William E Russell (1997) with modifications. Briefly, the livers were perfused through the portal vein with a calcium-free solution consisting of 150 mM NaCl, 2.8 mM KCl, 5.5 mM glucose, and 25 mM HEPES (pH 7.6) for 10 min, followed by mincing and digestion in DMEM containing 0.05% collagenase type IV for 30 minutes. The cells were centrifuged (2000g, 10minutes) and dispersed in DMEM enriched with 10% fetal Bovine Serum, 1% antibiotic-antimycotic.

### Primary culture of neonatal rat skeletal muscle

The skeletal muscles from 2-day-old neonatal rat were isolated by method described previously by William E Russell (1997) with modifications. Briefly, Soleus muscles were dissected, minced and digested with 0.2% Type II Collagenase and 0.05% trypsin in Phosphate buffered Saline (PBS) pH 7.4, 0.15M NaCl. The dispersed skeletal muscle cells were centrifuged (1000g, 10minutes) washed and resuspended in Phosphate buffered Saline (PBS) with 1% antibiotic-antimycotic. Cells were preincubated for 30 minutes at 37°C, 95%air/5%CO2. The floating muscle cells were plated on collagen coated T-25 flaks in Dulbecco's modified Eagle Medium enriched with 10% Fetal Bovine Serum and 1% antibiotic-antimycotic.

### Example 12

Viability of PC-3 after treatment as at the indicated times was determined by MTT assay -Cell titre 96 *AQ*oueus One solution Cell Proliferation Assay (Promega,Corp.Madison,WI) as per manufacturer's protocol. Briefly, 10,000 cells were plated in triplicate in 96 well plates and incubated for 12 hours in complete media. Compound MK-3 was added as indicated on replacing them to fresh complete media and incubated upto 72hours for varied time periods. 20µl per well of cell titre 96 *AQ*oueus One solution reagent. Incubate the plate for 1-4 hours at 37°C and humidified 5%CO₂ atmosphere. Absorbance was recorded at 490nm with 96 well plate readers. Reference wavelength of 630nm was used to reduce background contributed by nonspecific absorbance due to cell debris.

### Example 13

### Electrophonesis and Immunobloting

PC-3 cells (1X10⁶ cells) were incubated with complete medium alone or with 3µg/ml of Mahanine (MK-3) as indicated. Cells were detached using cell dissociation reagent from Sigma Chemical company St Louis,MO,USA. They were collected by centrifugation at 1500g for 10 mins and boiled for 5-7 minutes in sodium dodecyl sulphate(SDS) buffer (pH 6.8). Aliquots containing 60µg total cellular protein were separated by 10%SDS-PAGE and transferred to PVDF membrane (MILIPORE, Bedford, MA, USA). Membrane was blocked with blocking buffer for 1hour at room temperature and probed with desired primary antibody caspase-9, Bcl-xl, pAkt1/2/3(ser 473), anti PARP, Cytochrome C (Santa-Cruz Biotechnology, Inc. USA). Anti Caspase -3 that recognizes procaspse-3 (32 kDa) and the active cleaved caspase-3 (17 kDa) (BD Biosciences, Mountainview, CA). Akt, cleaved caspase-9 (Cell Signaling technology, Beverly,MA) overnight at 4° C followed by alkaline phosphatase conjugated secondary antibody and detection.

### Example 14

### Immunofluorescence

Cells were cultured on glass cover slips. Both control and treated cells were fixed with PBS containing 4% paraformaldehyde for 2h at 4° C. The cells were permiabilized with 0.1% Triton X-100 in PBS and then incubated with rabbit polyclonal anti-cytochrome c antibody (Santa cruz, USA, dil 1:50) for 3 h followed by incubation with FITC-conjugated secondary antibody (goat anti rabbit, Santa Cruz, 1:50) for another 1h with rigorous washing in all the above steps with PBS. 1µg/ml DAPI was also added in each set. The stained cells were observed under fluorescence microscope (Olympus BX51 microscope, Tokyo, Japan) and the images were captured with cool Snap Pro camera.

### Mahanine reduces PC-3 cell viability by inducing Apoptosis

Mahanine, purified from HPLC fraction of Murraya koenigii extract, induced death of PC-3 cells. Chemical structure was then determined by using 2D NMR and Mass Spectra and found it to be a mahanine, whose structure was, reported earlier (TianshungWu, 1991 Phytochemistry)(fig 1).

Mahanine killed androgen independent cell line PC-3 and androgen dependent cell line LNCaP cells in a dose and time dependent manner. (Fig 2).

To evaluate the specificity of mahanine, its effect on other cells in primary culture, such as, hepatocytes, cardiomyoctye and skeletal muscle was examined. Fig 3 shows that, mahanine did not kill other cells thereby suggesting its specificity for Prostate cancer cells.

### In-vivo experiment to confirm in-vitro results

Experiments for acute toxicity are performed as per the guidelines of WHO. Experimental subject: Mice, Balb/C Male, female. 25gms body weight (approx) 16 mice were divided into 2 groups 8 animals were received the compound @2gm/kg body weight. Equal volume of the vehicle (DMSO) were given to 8 animals wherein dose administered orally once and then animals sacrificed and haematological and biochemical parameters checked 15 days after treatment.

**Table 2(a): Haematological tests for Compound mahanine:**

| | RBC | WBC | Hb(gm%dl) |
|---|---|---|---|
| Control | 1029 | 92 | 10.69 |
| Treated | 1032 | 89 | 10.713 |

Biochemical parameters were assessed for the pure molecule mahanine. Since it is a pure molecule there is a need to see the biochemical profile, therefore we concentrated on the biochemical parameters only. The experimental procedure followed was same as that for combined extract..

**Table 2(b): Biochemical tests**

| | SGOT(units/ml) | SGPT(units/ml) | Urea(mg/dl) | Creatinine(mgldl) |
|---|---|---|---|---|
| Control | 147.5 | 32.0 | 35.4 | 3.82 |
| Treated | 174.0 | 30.7 | 48.6 | 3.72 |

Moreover there was no indication of any toxicity in mahanine treated mice (table 2(a),(b).

### Example 15

### Inhibition of cell survival pathway in mahanine treated PC-3 cells

In cell survival pathway, Akt kinase plays an important role by inhibiting apoptotic processes. Akt phosphorylates downstream effector molecules such as pro-apoptotic protein Bad effecting its inactivation. This does not permit its dimerisation with Bcl-XL that result in the inhibition of apoptotic process. Mahanine inhibited phosphorylation of Akt in a dose and time dependent manner. Mahanine showed no effect on the steady state levels of Akt protein but its serine 473 phosphorylation was inhibited at 24 h and by 36 h its activation was totally blocked (Fig 4).

The basal level of phosphorylation at serine 473 is predominant in PC-3 cell line The inactivation of Akt phosphorylation could be attributed to the inactivation of cell survival pathways resulting subsequent induction of apoptosis in PC-3 cell treated with mahanine.

We then investigated whether mahanine induced apoptosis of PC-3 cells is effected through Bcl-xl. Bcl-xl is a mitochondrial membrane protein that maintains mitochondrial membrane integrity in survival signal pathway. Mahanine significantly decreased Bcl-xl expression at 36 h (Fig 5).

This indicates initiation of apoptotic pathway as down regulation of Bcl-xl permits disintegration of mitochondrial outer membrane that causes leakage of cytochrome c initiating the caspase cascade. Mahanine causes leakage of cytochrome c from the mitochondria in PC-3 cells Caspase 9 activation was determined in the same time points with the same dose (3 µg/ml of culture media) of mahanine. Cleaved caspase 9 could be detected as early as 6h which steadily increased at 12h and 24h and declined at 36h (Fig 6a). The activation of caspase 3 considerably increased at 48h and peak of the activity could be detected at 60h (Fig 6b).

Mahanine effect on caspase 9 and Caspase 3 indicates the activation of death pathway and time of activation of caspase 9 synchronized with caspase 3 which is further downstream in the apoptotic pathway.

Poly ADP ribosyl Polymerase (PARP) is a DNA repair enzyme responsible for nick detection and repair .It is one of the target proteins of caspase 3. Active caspase 3 causes cleavage of PARP into an 89kDa fragment in PC-3 cells due to mahanine. The maximum cleavage of PARP was observed at 36h (fig 7).

In the light of the above results it may be concluded that mahanine effectively induces apoptosis of both androgen dependent and androgen independent) prostate cancer cells,

### Advantages:

### Described herein are inter alia ;

1. A simplified method of bioactive extraction and a simplified fast and inexpensive process for the preparation of fraction mahanine possessing significantly high biological activities relevant to treatment, relief and remedy of prostate cancer.
2. A pharmaceutical composition which is highly compatible for human consumption and capable for being used for the treatment, relief and remedy of prostate cancer.

## Claims

1. Use of a composition comprising a therapeutically effective amount of compound mahanine or pharmaceutically acceptable salt thereof optionally along with one or more pharmaceutically acceptable carriers in the manufacture of a medicament for the treatment of prostate cancer.

2. Use according to claim 1, wherein the mahanine used is obtained from the extract of *Murrya Koenigii* or synthetically made.

3. Use according to claim 1, wherein the dosage of the said composition is administered in water-soluble form at a unit dose of at least 0.1-5.0mg/kg body weight.

4. Use according to claim 1, wherein the dosage of the said composition is administered preferably in water-soluble form.

5. Use according to claim 1, wherein the said carriers is selected from the group consisting of proteins, carbohydrates, sugar, magnesium stearate, cellulose, calcium carbonate, and starch-gelatin paste and pharmaceutical acceptable carriers, excipient, diluent or solvent.

6. Use according to claim 1, wherein the administration route is selected from the group consisting of, oral, intravenous, intramuscular or subcutaneous route.

7. Use according to claim 6, wherein the said form for oral route is selected from the group consisting of capsule, syrup, concentrate, powder and granules.

8. Use according to claim 1, wherein the androgen independent cell line PC 3 and androgen dependent cell line LNCaP is killed by the said composition in a dose and time dependent manner.

9. Use according to claim 8, wherein the phosphorylation of Akt is inhibited by the said composition in a dose and time dependent manner.

10. Mahanine for use in the treatment of prostate cancer.

## Patentansprüche

1. Verwendung einer Zusammensetzung, umfassend eine therapeutisch wirksame Menge der Verbindung Mahanin oder eines pharmazeutisch verträglichen Salzes davon, optional zusammen mit einem oder mehreren pharmazeutisch verträglichen Trägern, bei der Herstellung eines Medikaments zur Behandlung von Prostatakrebs.

2. Verwendung nach Anspruch 1, worin das verwendete Mehanin aus dem Extrakt von *Murrya Koenigii* erhalten wird oder synthetisch hergestellt wird.

3. Verwendung nach Anspruch 1, worin die Dosis der Zusammensetzung in einer wasserlöslichen Form in einer Einheitsdosis von mindestens 0,1 - 5,0 mg/kg Körpergewicht verabreicht wird.

4. Verwendung nach Anspruch 1, worin die Dosis der Zusammensetzung vorzugsweise in wasserlöslicher Form verabreicht wird.

5. Verwendung nach Anspruch 1, worin die Träger ausgewählt werden aus der Gruppe, bestehend aus Proteinen, Kohlenhydraten, Zucker, Magnesiumstearat, Cellulose, Calciumcarbonat und Stärke-Gelatine-Paste und pharmazeutisch verträglichen Trägern, pharmazeutisch verträglichem Exzipient, Verdünnungsmittel oder Lösungsmittel.

6. Verwendung nach Anspruch 1, worin der Verabreichungsweg ausgewählt wird aus der Gruppe, bestehend aus oralem, intravenösem, intramuskulärem oder subkutanem Weg.

7. Verwendung nach Anspruch 6, worin die Form für den oralen Weg ausgewählt wird aus der Gruppe, bestehend aus Kapsel, Sirup, Konzentrat, Pulver und Granalien.

8. Verwendung nach Anspruch 1, worin die Androgen-unabhängige Zelllinie PC 3 und Androgen-abhängige Zelllinie LNCaP durch die Zusammensetzung in einer Dosis- und Zeit-abhängigen Art abgetötet wird.

9. Verwendung nach Anspruch 8, worin die Phosphorylierung von Akt durch die Zusammensetzung in einer Dosis- und Zeit-abhängigen Art inhibiert wird.

10. Mahanin zur Verwendung bei der Behandlung von Prostatakrebs.

## Revendications

1. Utilisation d'une composition comprenant une quantité thérapeutiquement efficace du composé mahanine ou un sel pharmaceutiquement acceptable de celui-ci optionnellement avec un ou plusieurs supports pharmaceutiquement acceptables dans la fabrication d'un médicament pour le traitement du cancer de la prostate.

2. Utilisation selon la revendication 1, où la mahanine utilisée est obtenue de l'extrait de *Murrya Koenigii* ou est réalisée de manière synthétique.

3. Utilisation selon la revendication 1, où le dosage de ladite composition est administré sous forme soluble dans l'eau à une dose unitaire d'au moins 0,1-5,0mg/kg de poids corporel.

4. Utilisation selon la revendication 1, où le dosage de ladite composition est administré de préférence sous forme soluble dans l'eau.

5. Utilisation selon la revendication 1, où lesdits supports sont sélectionnés dans le groupe consistant en protéines, hydrates de carbone, sucre, magnésium stéarate, cellulose, calcium carbonate et pâte amidon-gélatine et des supports pharmaceutiquement acceptables, excipient, diluant ou solvant.

6. Utilisation selon la revendication 1, où la voie d'administration est sélectionnée dans le groupe consistant en voie orale, intraveineuse, intramusculaire ou sous-cutanée.

7. Utilisation selon la revendication 6, où ladite forme de voie orale est sélectionnée dans le groupe consistant en capsule, sirop, concentrés, poudre et granulés.

8. Utilisation selon la revendication 1, où la lignée cellulaire PC 3 androgéno-indépendante et la lignée cellulaire LNCaP androgéno-dépendante est tuée par ladite composition d'une manière dépendant de la dose et du temps.

9. Utilisation selon la revendication 8, où la phosphorylation de Akt est inhibée par ladite composition d'une manière dépendant de la dose et du temps.

10. Mahanine pour utilisation dans le traitement du cancer de la prostate.
